# EUROPEAN PATENT SPECIFICATION

(11) **EP 0 772 579 B1**
(45) Date of publication and mention of the grant of the patent: **10.03.1999**
(21) Application number: 95926689.1
(22) Date of filing: 14.07.1995
(51) Int. Cl.: C07C 47/575, C07C 49/84, C07C 45/45, C07C 45/29, C07C 45/51

(54) **BENZOYL DERIVATIVES AND SYNTHESIS THEREOF**
BENZOYLDERIVATE UND IHRE HERSTELLUNG
DERIVES DE BENZOYLE ET LEUR SYNTHESE

(30) Priority: 20.07.1994 US 277724
(43) Date of publication of application: 14.05.1997
(73) Proprietor: MONSANTO COMPANY, St. Louis, Missouri 63167 (US)
(72) Inventor: HAMPER, Bruce, Cameron, Kirkwood, MO 63122 (US); LESCHINSKY, Kindrick, Lee, Ellisville, MO 63011 (US)
(74) Representative: Bosch, Henry
(86) International application number: US9508839
(87) International publication number: WO9602485

(56) References cited:
- CHEMICAL ABSTRACTS, vol. 122, no. 21, 22 May 1995 Columbus, Ohio, US; abstract no. 265364, NAKANISHI H ET AL 'Preparation of 4-phenyloxazole and -thiazole derivatives as herbicides' & JP,A,94 340 643 (NIHON NOHYAKU CO LTD;JAPAN) 13 December 1994
- CHEMICAL ABSTRACTS, vol. 119, no. 3, 19 July 1993 Columbus, Ohio, US; abstract no. 027827, TAKAISHI H ET AL 'Preparation of 3-hydroxyacetophenone derivatives and their intermediates' & JP,A,92 356 438 (NIHON NOHYAKU CO., LTD.;JAPAN) 10 December 1992
- J. CHEM. SOC., PERKIN TRANS. 1 (JCPRB4);74; (15); PP.1769-71, LA TROBE UNIV.;DEP. ORG. CHEM.; BUNDOORA; AUST., BROXTON T J ET AL 'Ring bromination of aryl methyl ketones with hypobromous acid'
- HETEROCYCLES (HTCYAM,03855414);85; VOL.23 (11); PP.2829-33, UNIV. TASMANIA;CHEM. DEP.; TASMANIA; 7001; AUSTRALIA (AU), BLACKMAN A J ET AL 'Amathamide alkaloids from the marine bryozoan Amathia Wilsoni Kirkpatrick'
- CHEMISCHE BERICHTE, vol. 47, 1914 WEINHEIM DE, pages 3040-3052, P. FRIEDLAENDER ET AL. 'Uber Hydroxylderivate des Indigblaus'

## Description

The present invention relates to novel benzoyl derivatives and their methods of manufacture. These compounds are useful for the preparation of agricultural chemicals and medicines and particularly, as intermediates for an active class of arylhaloalkylpyrazole and aryl alkylsulfonylpyrazole herbicides.

### BACKGROUND OF THE INVENTION

In recent years it has been found that one class of active herbicides are the substituted phenylpyrazoles, the phenyl and pyrazole moieties of which contain a variety of substituents.

Methods of manufacturing these phenylpyrazoles commonly involve chemical conversions of one or more radicals substituted on the phenyl and/or pyrazole moieties, e.g., by halogenation, esterification, etc. It is also known to prepare these compounds from substituted acetophenones by interaction with various compounds, including various esters which contribute the desired substituent radical to the 5-position of the pyrazole radical via cyclization of an intermediate phenyl diketone. For example, various halo- and/or alkyl-substituted acetophenones have been reacted with (halo)acetic acid esters to produce the corresponding phenyl diketone which is cyclized with hydrazine to yield phenylpyrazoles substituted in the 5-position of the pyrazole radical with (halo)alkyl groups.

It has recently been disclosed that certain 3-substituted aryl-5-substituted pyrazoles are particularly useful for broadspectrum control of a variety of weeds at very low application rates in a number of agronomically important crops. The aryl group is typically the phenyl radical substituted with halogen, alkyl, alkoxy and ester groups, substituents which are also commonly found on the pyrazole moiety. Particularly effective within this class of compounds are esters of 2-chloro-5-(4-halo-1-methyl-5-(trifluoromethyl)-1H-pyrazol-3-yl)-4-fluorobenzoic acid. These particular compounds are most readily available from 2-fluoro-5-alkylacetophenones and their derivatives. The literature, however, does not provide methods of preparation of these intermediates or related compounds that could provide the desired pyrazolylbenzoic acid esters. Thus, there is a need in the art for the discovery of novel intermediates and for efficient methods for the preparation of these substituted arylpyrazole compounds.

The present invention describes intermediates useful for production of compounds within this new class of herbicides.

### SUMMARY OF THE INVENTION

The present invention relates to a class of benzoyl derivatives of Formula I and synthesis methods therefor: 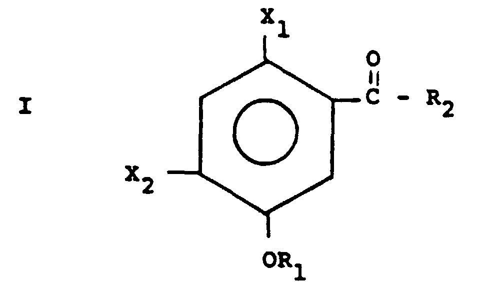 wherein X₁ is fluoro, X₂ is chloro, R₁ is methyl; and R₂ is C₁₋₆ alkyl or C₁₋₆ haloalkyl, H or -CH₂COR₃; wherein R₃ is a C₁₋₆ haloalkyl group.

A preferred subgenus of the benzoyl compounds in this invention are those according to Formula I wherein:
X₁ is fluorine;
X₂ is Cl;
R₁ is methyl;
R₂ is H, methyl or -CH₂COR₃ and
R₃ is CF₃, CF₂Cl, CF₂H or C₂F₅.

The most preferred species herein are those according to Formula I wherein X₁ is fluoro, X₂ is chloro, R₁ is CH₃ and R₂ is -CH₂COCF₃.

As readily apparent to those skilled in the art, when R₂ in Formula I is hydrogen, the resulting compound (Formula IA below) is a substituted benzaldehyde; when R₂ is methyl (Formula IB below), the compound is a substituted acetophenone and when R₂ is the -CH₂COR₃ radical, the resulting compound is a substituted phenyldiketone (Formula IC below). All of these compounds have the substituted benzoyl radical as a common structural feature, hence, for simplicity and convenience herein all of these compounds will be referred to collectively as benzoyl derivatives.

To applicants' knowledge all of the substituted benzoyl derivatives herein are novel compounds.

### DETAILED DESCRIPTION OF THE INVENTION

Compounds of Formula I wherein R₁ is a methyl group, X₁ is fluoro, X₂ is H or a halogen and R₂ is H (Formula IA) or methyl (Formula IB) are prepared from 2-substituted-4-fluoroanisoles of Formula II, which are known in the art, according to the following equation

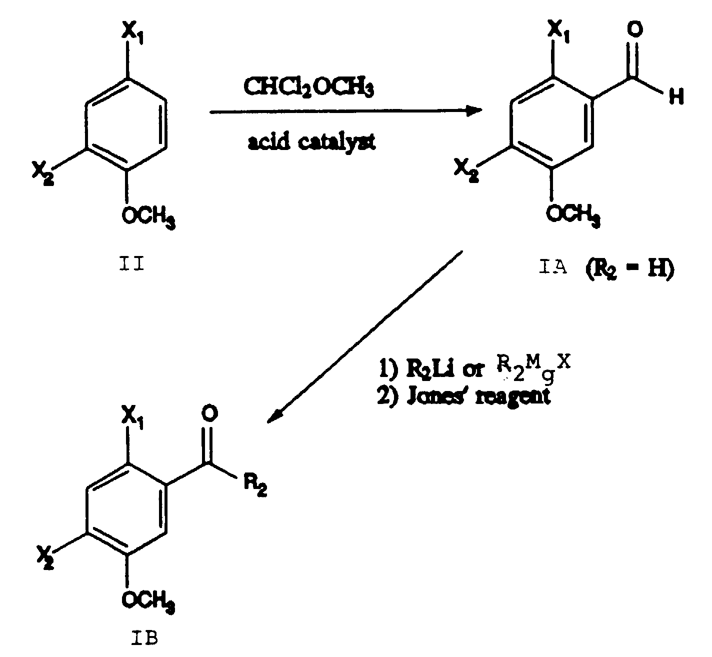

Typically, 2,4-dihalo-5-alkoxybenzaldehydes of Formula IA are prepared by alkylation of 2,4-dihaloanisoles of Formula II with a 1,1-dihaloalkylalkylether in the presence of an acid catalyst at a temperature in the range of -100°C to 100°C, preferably -78°C to 50°C. An alkylating agent such as 1,1-dichloromethyl methyl ether is preferred and can be employed in a range of one molar equivalent to an excess. The acid catalyst may be a Lewis acid such as TiCl₄, SnCl₄, FeCl₃ or a Bronstead acid such as H₂SO₄. The amount of catalyst can be from less than 0.1 mole % to excesses greater than 100 mole % relative to the 2,4-dihaloanisole. Any inert solvent may be used in this reaction that does not markedly hinder the reaction from proceeding or the reaction can be carried out neat. Preferred solvents include, but are not limited to, dichloromethane, dichloroethanes, nitrobenzene or hydrocarbons. Products are isolated by treatment of the reaction mixture with water and isolation of the product by standard methods. Yields of the desired materials can be favorably improved by treatment of the crude product with mineral acid, such as conc. H2SO4 or HCl to convert any geminal dichlorides to aldehydes. Isolation can then be caried out in the usual manner.

Acetophenones and alkyl aryl ketones of Formula I wherein R₂ is a lower alkyl group can be prepared from the above obtained benzaldehydes by a sequence of known reaction types. The benzaldehyde is treated with an organometallic reagent such as an alkyl lithium or alkyl Grignard reagent to give an intermediate benzyl alcohol. Methyl lithium and methyl Grignard are preferred for preparation of the acetophenones. The reactions can be carried out in any suitable anhydrous solvent such as THF, diethyl ether, toluene. Oxidation of the benzyl alcohol with any suitable oxidizing agent gives the desired aryl alkyl ketone of Formula I. Preferred oxidants include, but are not limited to, chromium oxide, chromium oxide in sulfuric acid, potassium permanganate, potassium dichromate, etc. Reaction temperature is in the range of -78°C to the boiling point of the inert solvent, preferably 0°C to 100°C. The reaction period may be chosen from the range of a few minutes to several weeks depending on the amounts of reagents, reaction temperature, etc.

Compounds of Formula IC are prepared from compounds of Formula IB by reaction with R₃COZ wherein Z is a C₁₋₆ alkoxy or C₆₋₈ aryloxy group or a halogen atom or by reaction with anhydride (R₃CO)₂O, where in both formulae R₃ is C₁₋₆ haloalkyl.

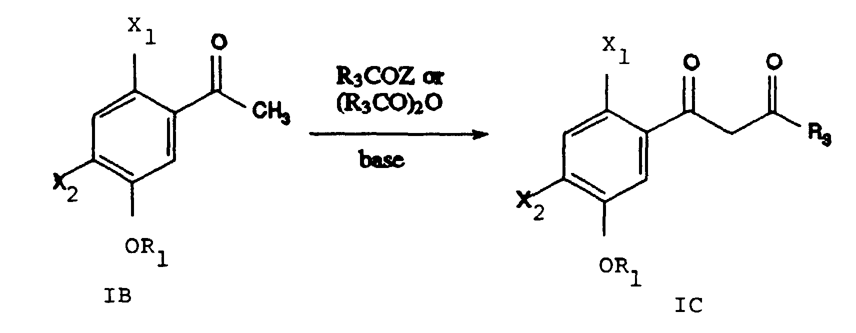

Thus, diketones of Formula IC can be prepared by treatment of 2-fluoro-4-(H or halogen)-5-alkylacetophenones with an ester, an anhydride or an acid halide in the presence of a base. Any suitable solvent or mixture of solvents can be employed; the preferred solvents are anhydrous ether, alcohols, dimethylsulfoxide, toluene, benzene, etc. The reaction is carried out in the presence of a base such as an alkali alkoxide, alkali amide or alkali hydride with the alkali alkoxides such as sodium methoxide being preferred. Reaction temperature is in the range of -100°C to 200°C, preferably -78°C to the reflux temperature of the solvent. The reaction period may be chosen from the range of a few minutes to several weeks depending on the amounts of reagents, reaction temperature, etc.

Compounds of Formula IC are meant to include all possible tautomers, such as enols and all possible salts wherein the cation is an alkali metal or other suitable organic or inorganic cationic species.

The compounds of Formula IC can be converted to pyrazolylbenzoyl esters useful as synthetic herbicides by the following reactions.

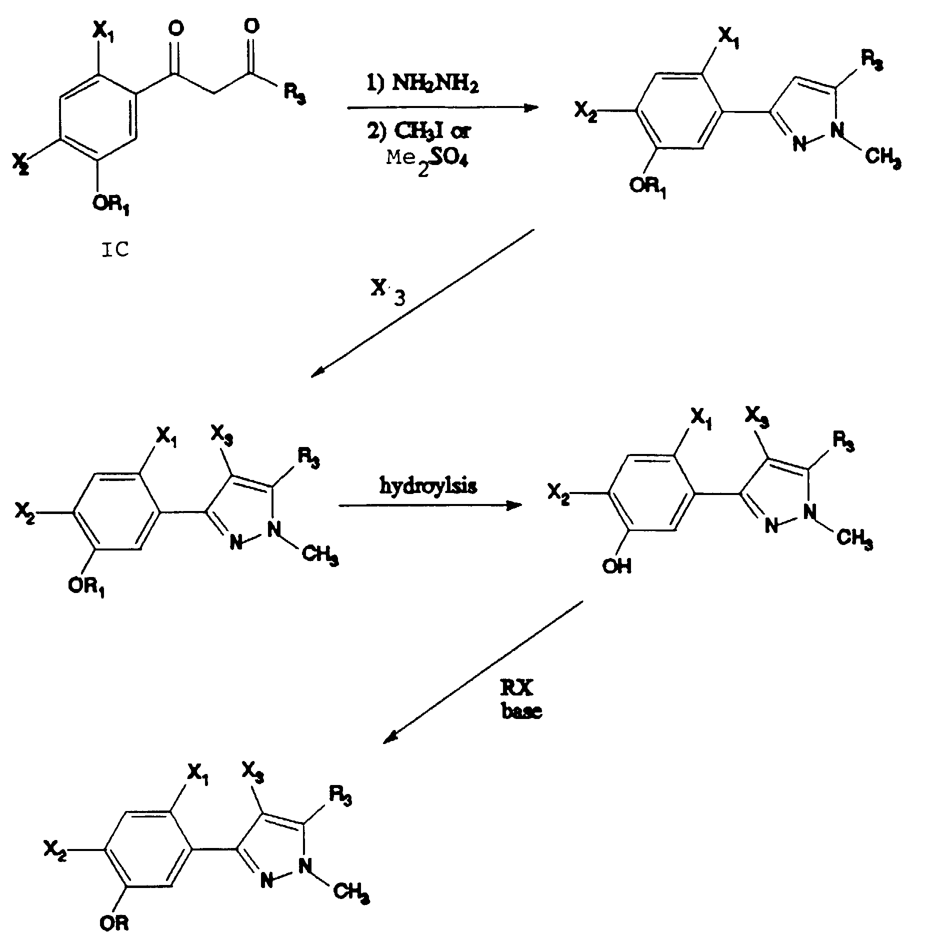

In the above formulae, R₁, R₃, X₁ and X₂ are as previously defined for Formula I, X₃ is halogen and R is an alkyl or substituted alkyl group.

The following Examples 1-9 describe specific working embodiments for the preparation of representative compounds according to this invention.

### EXAMPLE 1

Preparation of 4-Chloro-2-fluoro-5-methoxybenzaldehyde.

To a nitrogen purged 3 L round bottom flask equipped with a mechanical stirrer and a gas scrubber was added 114 g of titanium(IV) chloride followed by 48 g of 2-chloro-4-fluoroanisole. The stirred mixture was cooled in an ice water bath and treated dropwise with of 68.4 g of α,α-dichloromethyl methyl ether. After stirring for 90 minutes, the resultant slurry was treated with 200 mL of methylene chloride and the reaction allowed to reach room temperature. The mixture was treated with an additional 500 mL of methylene chloride, added dropwise to ice water in a 4 L beaker and the resultant mixture extracted three times with methylene chloride. The combined organic extracts were washed with water, 10% Na₂CO₃, dried and concd to give a crude oily solid. Trituration with hexanes yielded 42 g (74%) of 4-chloro-2-fluoro-5-methoxybenzaldehyde as a white solid. An analytical sample was obtained by bulb-to-bulb distillation to give a white, crystalline solid: mp 120.0-122.0°C; ¹H NMR (CDCl₃) δ 3.93 (s,3H), 7.25 (d, 1H, J = 9.4 Hz), 7.34 (d, 1H, 5.9 Hz), 10.28 (s, 1H).

| Anal. Calcd for C₈H₆O₂Cl₁F₁: | | | |
|---|---|---|---|
| | C, 50.95; | H, 3.21; | Cl, 18.80. |
| Found | C, 50.83; | H, 3.24; | Cl, 18.90. |

### EXAMPLE 2

Preparation of 1-(4-Chloro-2-fluoro-5-methoxyphenyl)ethanone.

A stirred solution of 10.4 g of 4-chloro-2-fluoro-5-methoxybenzaldehyde in 150 mL of anhydrous THF was cooled in a dry ice-acetone bath and treated with 35 mL of a 3M solution of methyl magnesium chloride in THF over a period of one minute. The ice bath was removed and the mixture allowed to warm to room temperature. After warming, the solution was slowly poured into ice water. The water slurry was extracted three times with diethyl ether, the ether extracts dried and concd to afford a crude oil. Crystallization from hexanes yielded 10.8 g (95.6%) of 4-chloro-2-fluoro-5-methoxy-α-methylbenzenemethanol: mp 68.5-69.5°C. This benzenemethanol intermediate was dissolved in 100 mL of acetone, cooled in an ice water bath and treated dropwise with 50 mL of freshly prepared Jones' reagent (prepared from 6.7 g of CrO₃, 6 mL of H₂SO₄ and 50 mL of water), keeping the temperature below 10°C. After stirring for 2 hrs, the solution was diluted with water and extracted three times with methylene chloride. The organic extracts were dried and concd to give a crude product. Recrystallization from methanol yielded 9.66 g (90.3%) of 1-(4-chloro-2-fluoro-5-methoxyphenyl)ethanone as a white solid: mp 96.5-98.5°C; ¹HNMR (CDCl₃) δ 2.50 (d, 3H, 5.4 Hz), 3.80 (s, 3H), 7.10 (d, 1H, 10.1 Hz), 7.30 (d, 1H, 6.3 Hz).

| Anal. calcd for C₉H₈O₂Cl₁F₁: | | |
|---|---|---|
| | C, 53.55; | H, 3.98. |
| Found | C, 53.45; | H, 3.96. |

### EXAMPLE 3

### Preparation of 1-(4-Chloro-2-fluoro-5-methoxyphenyl)-4,4,4-trifluoro-1,4-butanedione.

A solution of 21.8 g of 1-(4-chloro-2-fluoro-5-methoxyphenyl)ethanone in 100 ml of anhydrous diethyl ether was cooled in an ice bath. The stirred mixture was treated all at once with 28.1 g of ethyl trifluoroacetate. After stirring for a few minutes, 50 mL of 25% sodium methoxide in methanol (0.20 mol) was added and the solution was allowed to stir overnight. The mixture was quenched with 150 ml of water and 100 ml conc. HCL. The reaction mixture was extracted three times with diethyl ether and the combined organic layers separated, dried, and concd to afford a tan solid. The crude solid was recrystalized from methanol to give 23.5 g (73.2%) of 1-(4-chloro-2-fluoro-5-methoxyphenyl)-4,4,4-trifluoro-1,4-butanedione as a yellow solid: mp 122-123°C; ¹HNMR (CDCl₃) δ 3.80 (d, 3H, 2 Hz), 6.60 (d, 1H, 2 Hz), 7.10 (dd, 1H, 11 Hz, 2 Hz), 7.40 (dd, 1H, 4 Hz, 2 Hz).

| Anal. Calcd for C₁₁H₆O₃Cl₁F₄: | | |
|---|---|---|
| | C, 44.39; | H, 2.03. |
| Found | C, 44.23; | H, 2.36. |

Examples 4-6 were prepared by alkylation of the corresponding anisole in a manner analogous to the process of Example 1.

Example 7 was prepared according to the two-step addition-oxidation sequence in a manner analogous to the process of Example 2.

Examples 8 and 9 were prepared according to the process analogous to that in Example 3.

Physical properties for the compounds of Examples 4-9 are shown in the table below.

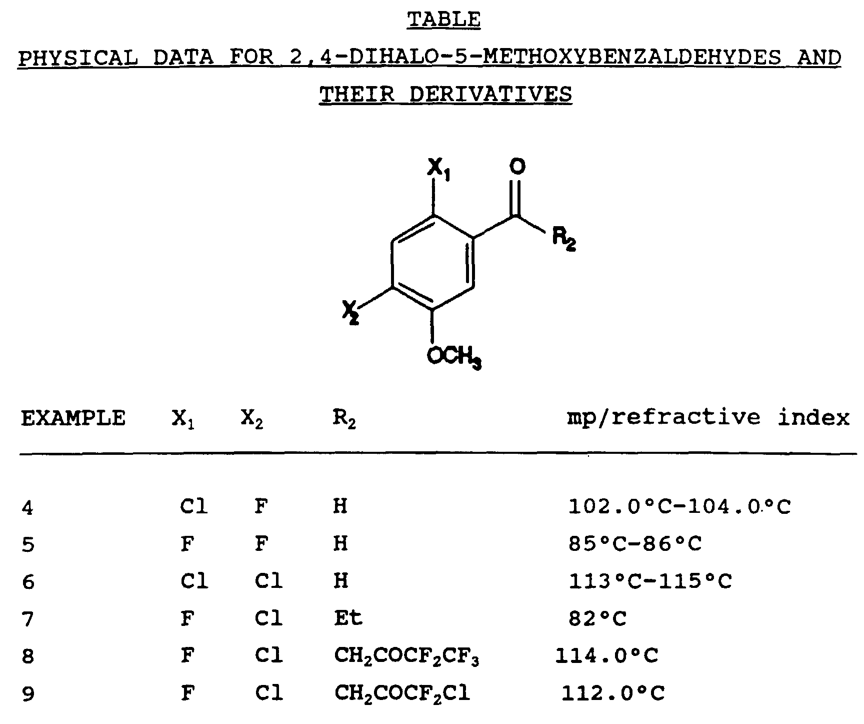

The novel 2,4-dihalo-5-alkoxybenzaldehydes, 2,4-dihalo-5-alkoxyacetophenones and benzoyl derivatives of the present invention are useful as intermediates for the preparation or manufacture of agricultural chemicals and medicines, particularly the substituted phenylpyrazole type herbicides. These intermediates allow direct introduction of a 5'-alkoxy substituent on the phenyl ring of the phenylpyrazole which can be converted to 5'-substituted oxyphenyl pyrazoles such as 5'-propargyloxyphenylpyrazoles or pyrazolylphenoxyacetic acids or esters.

As will be appreciated by those skilled in the art, various modifications of the invention described herein may be made without departing from the spirit and scope thereof.

## Claims

1. Compound according to Formula I 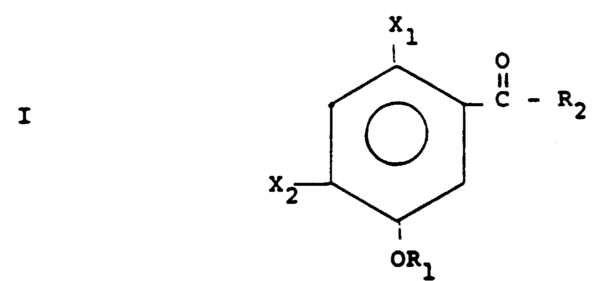 wherein
X₁ is fluoro;
X₂ is chloro
R₁ is methyl and
R₂ is H, C₁₋₆ alkyl or C₁₋₆ haloalkyl or -CH₂COR₃, wherein R₃ is C₁₋₆ haloalkyl.

2. Compound according to Claim 1 wherein R₂ is methyl.

3. Compound according to Claim 2 wherein R₃ is -CF₃.

4. Process for preparing compounds according to Formula IC 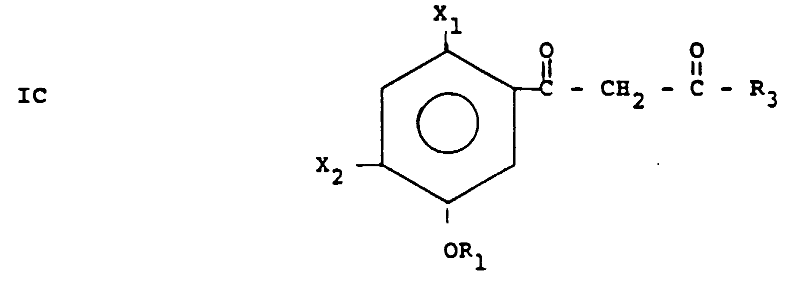 which comprises acylating a compound according to Formula IB 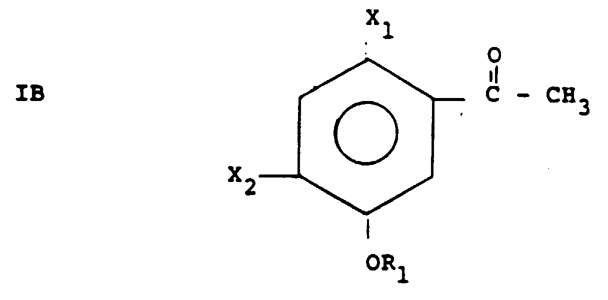 wherein
X₁ and X₂ are H or halogen;
R₁ is C₁₋₆ alkyl and
R₃ is C₁₋₆ haloalkyl,
with an ester, acetyl halide or anhydride in an inert solvent in the presence of a strong base and after workup recovering said compound according to Formula IC.

5. Process according to Claim 4 wherein X₁ is fluoro, X₂ is chloro, R₁ is methyl and R₃ is -CF₃.

6. Process according to Claim 4 wherein said compounds according to Formula IB are prepared by reacting a compound according to Formula II 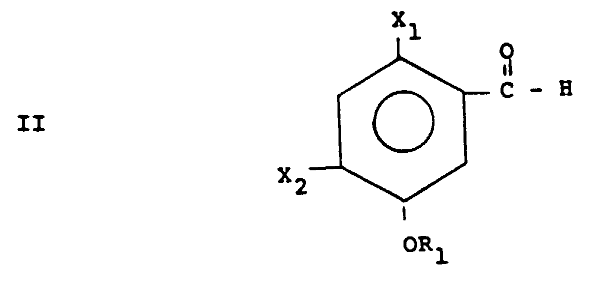 wherein X₁, X₂ and R₁ are as previously defined, with an organometallic reagent, followed by reaction with an oxidation agent in an inert solvent.

7. Process according to Claim 6 wherein said organometallic reagent is an alkyl lithium or alkyl Grignard reagent and said oxidation agent is Jones' reagent.

8. Process according to Claim 7 wherein said organic metallic reagent is methyl lithium or methyl Grignard.

9. Process for preparing compounds according to Formula IA 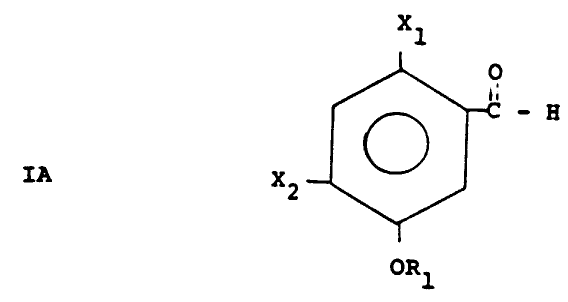 which comprises reacting a compound according to Formula II 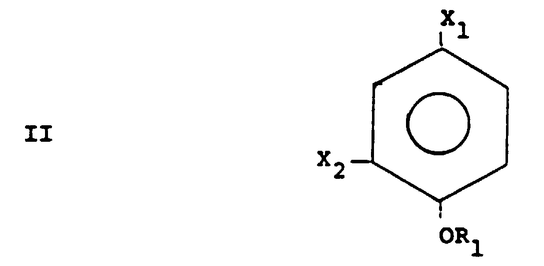 wherein X₁, X₂ and R₁ are as previously defined, with an alkylating agent in the presence of an alkylating catalyst.

10. Process according to Claim 9 wherein said alkylating agent is a halo C₁₋₄ alkyl C₁₋₄ alkyl ether and said catalyst is a Lewis or Bronstead Acid.

11. Process according to Claim 9 wherein X₁ is fluoro, X₂ is chloro, R₁ is methyl, said alkylating agent is dichloromethyl methyl ether and said catalyst is a metal halide.

## Patentansprüche

1. Verbindung der Formel (I) 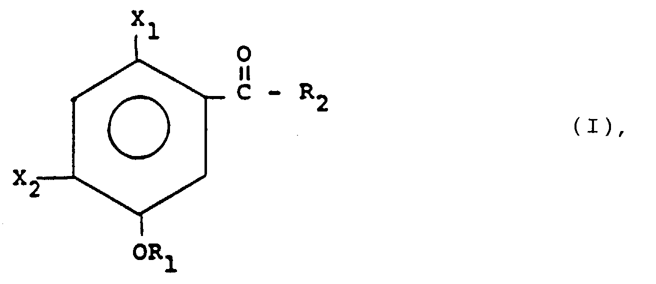 worin
X₁ Fluor bedeutet;
X₂ Chlor darstellt;
R₁ Methyl ist; und
R₂ die Bedeutung H, C₁-C₆-Alkyl oder C₁-C₆-Halogenalkyl oder -CH₂COR₃ hat, wobei R₃ C₁-C₆-Halogenalkyl darstellt.

2. Verbindung nach Anspruch 1, worin R₂ Methyl ist.

3. Verbindung nach Anspruch 2, worin R₃ die Bedeutung -CF₃ hat.

4. Verfahren zur Herstellung von Verbindungen der Formel (IC) 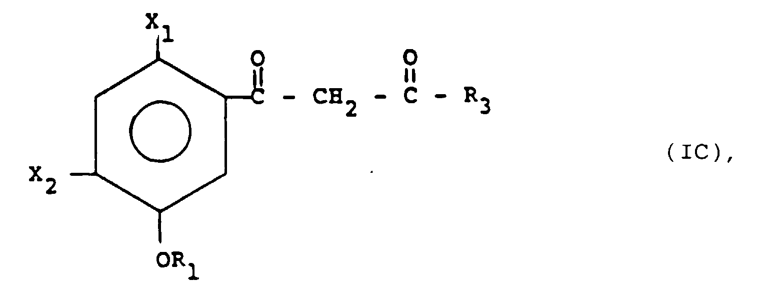 welches umfaßt: Acylieren einer Verbindung der Formel (IB) 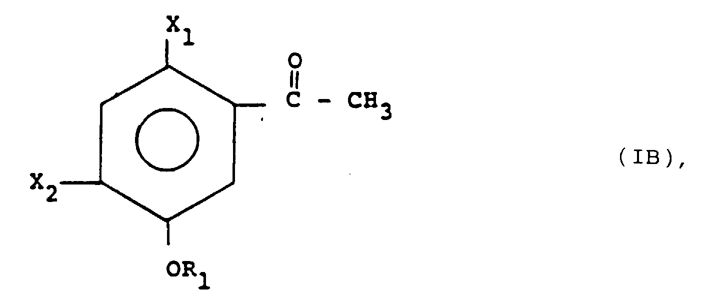 worin
X₁ und X₂ die Bedeutung H oder Halogen haben;
R₁ C₁-C₆-Alkyl darstellt; und
R₃ C₁-C₆-Halogenalkyl ist;
mit einem Ester, Acetylhalogenid oder Anhydrid in einem inerten Lösungsmittel in Anwesenheit einer starken Base, und nach Aufarbeiten Gewinnen der Verbindung der Formel (IC).

5. Verfahren nach Anspruch 4, wobei X₁ Fluor bedeutet; X₂ Chlor darstellt; R₁ Methyl ist; und R₃ die Bedeutung -CF₃ hat.

6. Verfahren nach Anspruch 4, bei welchem die Verbindungen der Formel (IB) hergestellt werden durch das Umsetzen einer Verbindung der Formel (II) 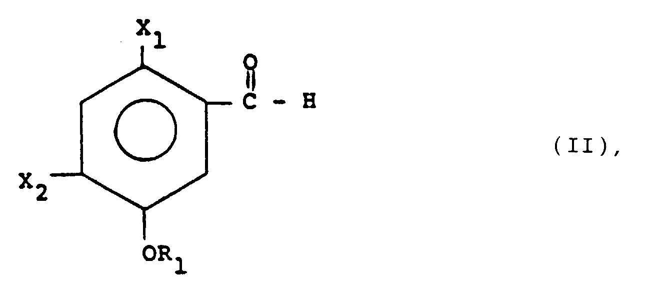 worin X₁, X₂ und R₁ wie vorstehend definiert sind, mit einem Organometall-Reagenz, gefolgt vom Umsetzen mit einem Oxidationsmittel in einem inerten Lösungsmittel.

7. Verfahren nach Anspruch 6, bei welchem das Organometall-Reagenz ein Alkyllithium- oder Alkyl-Grignard-Reagenz ist, und das Oxidationsmittel Jones-Reagenz ist.

8. Verfahren nach Anspruch 7, bei welchem das Organometall-Reagenz Methyllithium oder Methyl-Grignard ist.

9. Verfahren zur Herstellung von Verbindungen der Formel (IA) 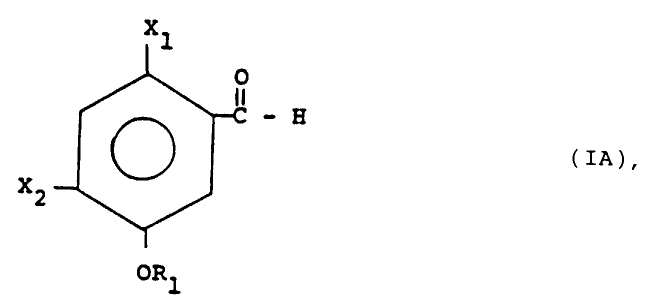 welches umfaßt: Umsetzen einer Verbindung der Formel (II) 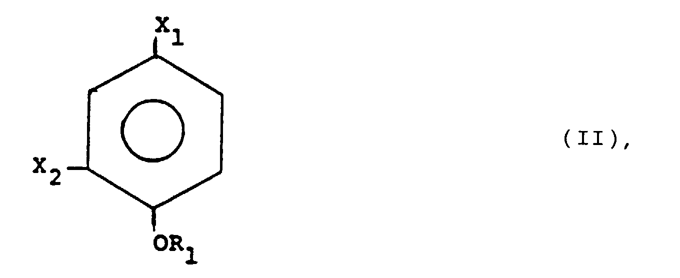 worin X₁, X₂ und R₁ wie vorstehend definiert sind, mit einem Alkylierungsmittel in Anwesenheit eines Alkylierungskatalysators.

10. Verfahren nach Anspruch 9, bei welchem das Alkylierungsmittel ein Halogen-C₁-C₄-alkyl-C₁-C₄-alkylether ist, und der Katalysator eine Lewis- oder Brønsted-Säure ist.

11. Verfahren nach Anspruch 9, wobei X₁ Fluor bedeutet, X₂ Chlor darstellt, R₁ Methyl ist, das Alkylierungsmittel Dichlormethylether ist, und der Katalysator ein Metallhalogenid ist.

## Revendications

1. Composé selon la formule I : 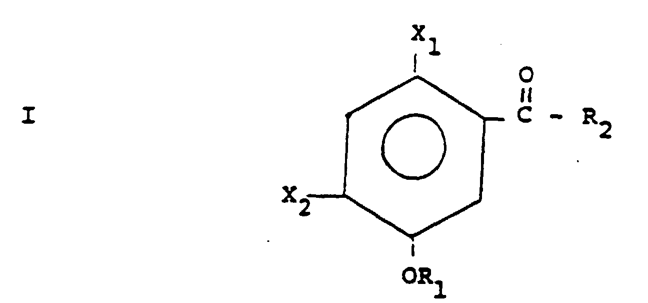 dans laquelle
X₁ est un fluoro,
X₂ est un chloro, R₁ est un méthyle, et
R₂ est H, un C₁₋₆ alkyle, un C₁₋₆ haloalkyle, ou -CH₂COR₃, où R₃ est un C₁₋₆ haloalkyle.

2. Composé selon la revendication 1, dans lequel R₂ est un méthyle.

3. Composé selon la revendication 2, dans lequel R₃ est -CF₃.

4. Procédé de préparation de composés ayant la formule IC : 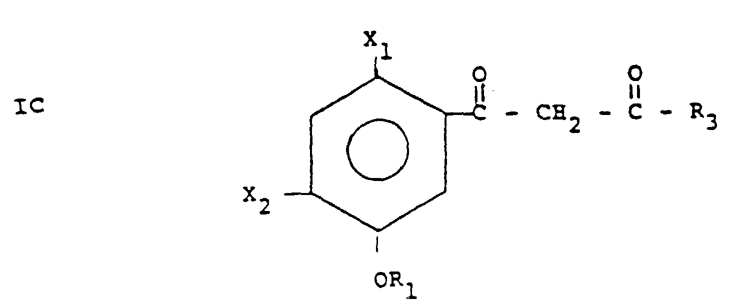 qui comprend d'acyler un composé ayant la formule IB : 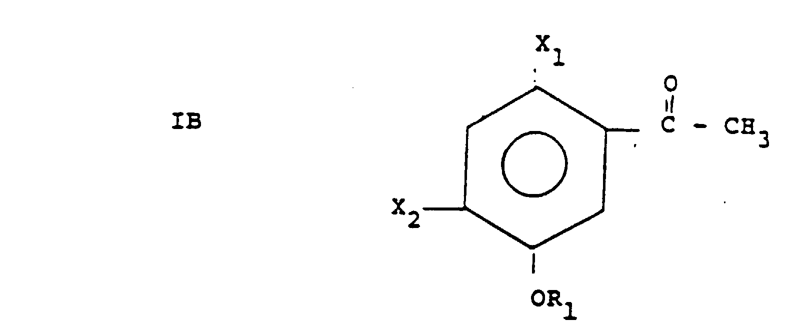 dans laquelle
X₁ et X₂ sont H ou un halogéno,
R₁ est un C₁₋₆ alkyle et
R₃ est un C₁₋₆ haloalkyle
avec un ester, un halogénure d'acétyle ou un anhydride, dans un solvant inerte, en présence d'une base forte, puis de récupérer et de purifier ledit composé ayant la formule IC.

5. Procédé selon la revendication 4, dans lequel X₁ est un fluoro, X₂ est un chloro, R₁ est un méthyle et R₃ est -CF₃.

6. Procédé selon la revendication 4, dans lequel lesdits composés ayant la formule IB sont préparés en faisant réagir un composé ayant la formule II : 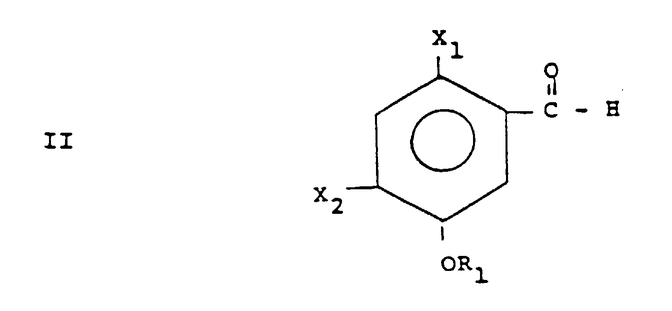 dans laquelle X₁, X₂ et R₁ sont comme défini précédemment, avec un réactif organométallique, la réaction étant suivie par une réaction avec un agent d'oxydation dans un solvant inerte.

7. Procédé selon la revendication 6, dans lequel ledit réactif organométallique est un alkyllithium ou un réactif de Grignard alkylé et ledit agent d'oxydation est le réactif de Jones.

8. Procédé selon la revendication 7, dans lequel ledit réactif organométallique est le méthyllithium ou le réactif de Grignard méthylé.

9. Procédé de préparation de composés ayant la formule IA 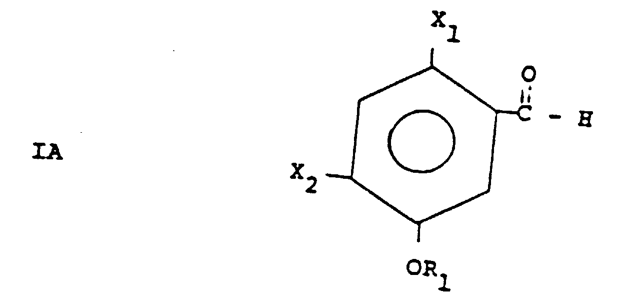 qui comprend de faire réagir un composé ayant la formule II 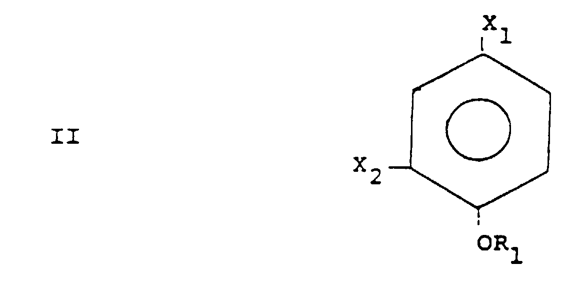 dans laquelle X₁, X₂ et R₁ sont comme défini ci-dessus, avec un agent d'alkylation, en présence d'un catalyseur d'alkylation.

10. Procédé selon la revendication 9, dans lequel ledit agent d'alkylation est un [halo C₁₋₄ alkyl][C₁₋₄ alkyl]éther et ledit catalyseur est un acide de Lewis ou un acide de Brönsted.

11. Procédé selon la revendication 9, dans lequel X₁ est un fluoro, X₂ est un chloro, R₁ est un méthyle, ledit agent d'alkylation est le dichlorométhylméthyléther et ledit catalyseur est un halogénure métallique.
